# EUROPEAN PATENT SPECIFICATION

(11) **EP 1 509 223 B1**
(45) Date of publication and mention of the grant of the patent: **20.08.2008**
(21) Application number: 03722906.9
(22) Date of filing: 09.05.2003
(51) Int. Cl.: A61K 31/352, A61P 1/16

(54) **HEPATOPROTECTIVE ACTIVITY OF 10-O-P-HYDROXYBENZOYIAUCUBIN**
LEBERSCHÜTZENDE WIRKUNG DER 10-O-P-HYDROXYBENZOYLAUCUBIN
ACTIVITE HEPATOPROTECTRICE DE LA 10-O-HYDROXYBENZOYLAUCUBINE

(30) Priority: 10.05.2002 US 397359 P
(43) Date of publication of application: 02.03.2005
(73) Proprietor: Council of Scientific and Industrial Research, 110 062 New Delhi (IN)
(72) Inventor: PRABHAKAR, Anil, 180 001 Jammu (IN); GUPTA, Bishan Datt, 180 001 Jammu (IN); SURI, Krishan Avtar, 180 001 Jammu (IN); SATTI, Naresh Kumar, 180 001 Jammu (IN); MALHOTRA, Swadesh, 180 001 Jammu (IN); JOHRI, Rakesh Kamal, 180 001 Jammu (IN); JAGGI, Bupinder Singh, 180 001 Jammu (IN); CHANDAN, Bal Krishan, 180 001 Jammu (IN); SHARMA, Ashok Kumar, 180 001 Jammu (IN); GUPTA, Devinder Kumar, 180 001 Jammu (IN); KAPAHI, Bal Krishan, 180 001 Jammu (IN); BEDI, Kasturi Lal, 180 001 Jammu (IN); SURI, Om Parkash, 180 001 Jammu (IN); QAZI, Gulam Nabi, 180 001 Jammu (IN)
(74) Representative: Petruzziello, Aldo
(86) International application number: PCT/IB2003/001810
(87) International publication number: WO 2003/094911

(56) References cited:
- CN-A- 1 200 290
- CN-A- 1 258 545
- CN-A- 1 264 595
- DUTTA P ET AL: "STUDIES ON INDIAN MEDICINAL PLANTS-PART LXXV. NISHINDASIDE, A NOVEL IRIDOID GLYCOSIDE FROM VITEX NEGUNDO" TETRAHEDRON (1983) 39 (19) P. 3067-3072., XP001154066 INDIAN INST OF CHEM BIOLOGY, CALCUTTA 700 032 INDIA
- HOBERG, E. ET AL.: "An analytical high performance liquid chromatographic method for the determination of agnuside and p-hydroxybenzoic acid contents in Agni-casti fructus" PHYTOCHEMICAL ANALYSIS, vol. 11, pages 327-329, XP002251551
- GÖRLER, K. ET AL.: "Iridoidführung von Vitex agnus-castus" PLANTA MEDICA, vol. 6, 1985, pages 530-531, XP002251552
- DAYRIT F M ET AL: "IDENTIFICATION OF FOUR IRIDOIDS IN THE PHARMACOLOGICALLY-ACTIVE FRACTION OF VITEX NEGUNDO, L" PHILIPPINE J SCI (1994) 123 (4) P. 293-304., XP002251549 PHILIPPINE INST PURE APPL CHEM, MANILA UNIV, QUEZON CITY MANILA
- HOUGHTON P J ET AL: "ANTI-HEPATOTOXIC ACTIVITY OF EXTRACTS AND CONSTITUENTS OF BUDDLEJA SPECIES" PLANTA MEDICA, THIEME, STUTTGART, DE, vol. 55, no. 2, 1989, pages 123-126, XP009012740 ISSN: 0032-0943
- OKUYAMA, E. ET AL.: "Pharmacologically active components of Viticis Fructus (Vitex rotundifolia). II. The components having analgesic effects" CHEM. PHARM. BULL, vol. 46, no. 4, pages 655-662, XP001154068
- KURUÜZÜM-UZ, A. ET AL.: "Glucosides from Vitex agnus-castus" PHYTOCHEMISTRY, XP002251550
- HÄNSEL, R. ET AL.: "Chemotaxonomische Untersuchungen in der Gattung Vitex L." PHYTOCHEMISTRY, vol. 4, 1965, pages 19-27, XP009015821

## Description

### Field of the invention

This invention relates to hepatoprotective activity of an iridoid glycoside, 10-O-p-hydroxybenzoylaucubin (agnuside) of the formula 1 isolated from *Vitex negundo* by extracting the aerial parts/whole plant with polar solvent like 95% ethanol, methanol, aqueous ethanol or water, removing fatty non polar constituents by triturating the extract with solvents such as ethylene chloride, methylene chloride, chloroform or ethyl acetate to get a fraction from which agnuside is separated by column chromatography. The hepatoprotective activity of agnuside has been confirmed by evaluation of its protective action against *CCl* and galactosamine induced liver damage models.

### Background and Prior art references

*Vitex negundo* Linn (family Verbenaceae) is widely used in the indigenous system of medicine in India. Various medicinal properties are ascribed to leaves and roots of this plant. The leaves are aromatic, tonic and vermifuge and the roots are used as expectorant, febrifuge and tonic.(Chopra, R.N., Nayar, S.L. and Chopra, I.C., Glossary of Indian Medicinal Plants, CSIR, New Delhi, 1956, p. 256; Wealth of India : Raw Material, CSIR, New Delhi, 1976, vol. X. p. 522) A number of compounds have been isolated from various parts of this plant From the leaves Ghosh and Krishna isolated a number of compounds viz. glucononitol, p-hydroxybenzoic acid, 5-hydroxyisophthalic acid and' 3, 4 - dihydroxybenzoic acid along with two glucosides and an amorphous alkaloid [Ghosh, T.P. and Krishna, S.,7. Indian Chem. Soc. 1936, 13, 634]. Masilungan reported the presence of the terpenes, oc-pinene, camphene, citral and β- caryophyllene in the essential oil of the leaves Masilungan, V.A. Philip. J. Sci. 1955, 84, 275]. A large number of flavonoids have been isolated from the leaves and twigs. These are casticin, orientin, isoorientin, luteolin, luteolin-7-0-glucoside, corymbosin, gardenins A and B, 3-0-desmethylartemetin,5-0-desmethylnobiletin,3',4',5,5',6,7,8-heptamethoxy-flavone, 3',5-dihydroxy-4',7,8-trimethoxyflavanone and 3',5-dihydroxy-4',6,7-trimethoxy flavanone [Sirait.L.M., Rimpler, H. and Haensal, R., Experientia 1962, 18, 72; Haensal, R.et al. Phytochemistry 1965,4 ,19; Banerji A. el al. Phytochemistry 1969, 8,511; Ferdous, A. J. et al. Bangladesh Acad.Sci. 1984, 8,23; Dayrit, F.M..et al. Philipp. J.Sci. 1987, 116, 403; Banerji, J. et al. Indian J.Chem 1988; 27B, 597; Achari, B. et al. Phytochemistry. 1984, 23, 703]. Stem-bark afforded five new flavone glycosides along with luteolin and acerosin. The new flavone glycosides are 6β - glucopyranosyl ― 7 - hydroxy-3',4',5',8-tetramethoxyflavone-5-O-a-L-rhamnopyranoside, 3', 7-dihydroxy-4',6, 8-trimethoxy flavone-5-O-(6"-O-acetyl-p-D-glucopyranoside),3,3',4',6,7-pentamethoxy flavone 5'-O-(4"-O-|3-D-glucopyranosyl-a-rhamnopyranoside, 4',5, 7-tri-hydroxyflavone-8-(2"-caffeoyl-a-glucopyranoside) and 3', 5,5',7-tetrahydroxy-4-methoxyflavone -3'-O-(4"-O-a-D- galactopyranosyl) galactopyranoside [Rao, V.K.et al. Indian J. Pharm. 1977,39, 41; Subramamian, P.M. and Misra, G.S. Indian J. Chem. 1978,16B, 615; Subramaniam, P.M. and Misra, G.S.J. Nat. Prod. 1979,42, 540]. A diterpenoid, 5 |3-hydro-8,11,13-abieta-trien-6a-ol and three triterpenoids, 2a, 3a-dihydroxyoleana-5 12- dien-28-oic acid, 2a, 3a-diacetoxyoleana-5, 12-dien-28-oic acid, 2,3 cc-diacetoxy-18-hydroxyoleana-5,12-dien-28-oic acid have been isolated from the seeds. These compounds exhibited anti inflammatory activity [Chawla, A.S., Sharma, A.K., Handa, S.S. and Dhar, K.L. Indian J. Chem. 1991, 30B, 773 and J.Nat. Prod. 1992,55,163]. Leaves, however, did not yield any triterpenoids but from the roots acetyloleanolic acid was isolated [Vishnol, S.P.,Shoeb, A., Kapil, R.S. and Popli, S.P. Phytochemistry 1983, 22, 597].
Five Iridoid glycosides have been reported from the leaves of *V*. *negundo.* These are aucubin, agnuside (Hansal et al. Phytochemistry 4, 1965, 9 negundoside 6'-p-hydroxybenzoyl mussaenosidic acid (Sehgal et al. Phytochemistry, 21, 1982, 363) and nishindaside (Datta et al. Tetrahedron 39,1983, 3067).

During our search for hepatoprotectives agents of plant origin, the aqueous alcoholic extract of *V*. *negundo* and a fraction isolated from it exhibited strong immuno-stimulating hepatoprotective activities. A process has been developed for the isolation of an immuno-stimulating agent from the leaves of *Vitex negundo* for which a patent has been granted to Regional Research Laboratory, Jammu [Suri, J.L. et. al Indian Patent No. 78388 dt. 19-03.97). Another patent application has been submitted by Regional Research Laboratory, Jammu for a process for isolation of a bioactive composition possessing hepatoprotective and immuno-stimulating activity (Application no. 16/DEL98 dt. 16.1.98) In view of the strong hepatoprotective activity exhibited by the iridoid glycosides of *Picrorhiza kurroa* (Ansari, R.A. et al Indian J. Med. Research, 1988, 87, 401) it was thought desirable to evaluate the iridoid glycosides of *V*. *negundo* for hepatoprotective activity. Agnuside, an iridoid glycoside, was isolated from *V*. *negundo* and evaluated for hepatoprotective activity along with the aqueous alcoholic extract of the plant. Both aqueous alcoholic extract (coded as 033) and agnuside (coded as 033 (1) showed marked hepatoprotective activity in experimentally induced hepatic damage with CCl₄ and galactosamine (GalN) in rats. A comparison with the known hepatoprotective agent silymarin revealed that 033 and 033 (1) exhibited higher hepatoprotective potential in most of the parameters with respect to their effect on elevated levels of serum and liver homogenate parameters (Table 1 and 2).

### Description of the invention

Thus the main objective of the present invention is to provide hepatopro tec live activity of a defined bioactive molecule isolated from leaves of *V. negundo* viz., agnuside of formula 1, as shown in the diagram accompanying this specifications.

Accordingly, the present invention provides the use of 10-O-p-hydroxybenzoylaucubin of claim 1, prepared by :
(a) powdering the plant material by known methods
(b) preparing the aqueous alcoholic extract by percolation
(c) concentrating the alcoholic extract by conventional method,
(d) removing fatty non polar constituents by triturating the extract with solvents such as ethylene chloride, methylene chloride, chloroform or ethyl acetate
(e) adsorbing the residue extract over silica gel,
(f) isolation of agnuside from the adsorbed extract by column chromatography and
(g) evaluating for hepatoprotective activity

The solvent used for extraction in step (b) is ethanol, aqueous ethanol, methanol, aqueous methanol or water.

The hepatoprotective activity of the compound is expressed in 50 mg/kg⁻¹ oral dose in rals which on extrapolation comes to be 300-4QO mg daily human dose (70 kg) in single or divided doses.

### Characterisation of agnuside 1

I obtained as crystalline compound, mp 148-50°C IvT : -466, UV-232 nm, IR (KBr) spectrum showed absorptions at 3400, 1700, 1642 and 1618 cm⁻¹. ¹H NMR (200 MHz, CD₃OD) 5 6.35 {dd, *J* 2,6, H-3) 5.12 (dd, *J*, 4,6, H-4) 2.70 (m-H-5) 4.48 (m, H-6) 5.82 (s, H-7) 2.94 (m, H-9) 5.05 (s, H-10) 4. 69 (d, *J* 8, H-1') 3.65 (m, H-2') 7.92 fdd, / 2,7,H-2 "6") 6.84 (dd, 7,2,7, H-3 ") ¹³CNMR δ 97.92(C-1), 141.50 (C-3), 105.35 (C-4), 46.04 (C-5) 82.58 (C-6) 132.09 (C-7), 142.55 (C-8), 46.04 (C-9) 63.46 (C-10), 100.07 (C-1') 74.56 (C-2'), 77.61 (C-3') 71.01 (C-4'). 77.82 (C-5') 62.49 (C-6') 121.81 (C-1") 132.73 (C-2",C-6"), 16.08 (C-3", 5"), 163.50 (C-4 "), 167.70 (CO)

### Detailed description of the Invention

Accordingly, the present invention provides the use of 10-0-p-hydroxybenzoylaucubin of claim 1. Another embodiment of the present invention wherein the said composition reduces the elevated levels of serum glutamin-pyruvic transaminase (GPT) about 70%.

In yet another embodiment of the present invention wherein the said composition reduces the elevated levels of serum glutamin-oxalo acetic transaminase (GOT) about 60%.

In still another embodiment of the present invention wherein the said composition reduces the elevated levels of serum alkaline phosphatase (ALP) about 62%.

In yet another embodiment of the present invention wherein the said composition reduces the elevated levels of serum tryglycerides about 70%.

In still another embodiment of the present invention wherein said composition against the elevated level of bilirubin about 72%.

In yet another embodiment of the present invention wherein compound agnuside is obtained from the whole plant.

In yet another embodiment of the present invention wherein 10-O-p-hydroxybenzoylaucubin is of concentration ranging between about 20 to 200 mg /kg-body weight.

In still another embodiment of the present invention wherein 10-O-p-hydroxybenzoylaucubin is of concentration is about 50 mg/kg-body weight.

In yet another embodiment of the present invention wherein the pathological condition is selected from liver disorder

In still another embodiment of the present invention wherein the subject is selected from mammals and animals preferably humans.

In yet another embodiment of the present invention wherein said composition is used singly or in combination with pharmaceutically acceptable carriers.

In still another embodiment of the present invention wherein said composition is administered to subject in combination with pharmaceutically acceptable additives, carriers, diluents, solvents, filters. Lubricants, excipients, binder or stabilizers.

In yet another embodiment of the present invention wherein the desired dosage is administered for both preventive and curative properties.

In still another embodiment of the present invention wherein said composition is administered, orally or by any clinically/medically accepted methods.

In yet another embodiment of the present invention wherein the preferred dosage for human beings is about 5 mg/Kg of body weight.

In still another embodiment of the present invention wherein the various physical forms in which the composition is available, e.g. powder, tablet, capsule, syrup, granules, emulsion, aerosoal, or beads.

In yet another embodiment of the present invention is useful for Liver cirrhosis, Galactosemia, Hemoanigoma, Hemochromatosis, Hepatitis A, Hepatitis B, Hepatitis C, Hepatitis D, Hepatitis E, Hepatitis G, Alcholic Liver disease, Autoimmune hepatitis, Cancer of Liver, Biliary Atresia, Glycogen Storage Disease 1, Alpha-1-antitrypsin deficiency, Alagille syndrome, Byler Disease, Caroli disease, Fatty liver, Itching in Liver, Primar Biliary Cirrhosis, Sclerosing Cholangitis or Protoporphyria Erythroepatic. Said compound 10-O-p-hydroxybenzoylaucubin compound of Formula 1, also called Agnuside, is isolated from aerial part/whole body by:
(a) powdering the plant material by known methods
(b) preparing the aqueous alcoholic extract by percolation
(c) concentrating the alcoholic extract by conventional method
(d) removing fatty non-polar constituents by triturating the extract with solvents such as ethylene chloride, methylene chloride, chloroform or ethyl acetate
(e) adsorbing the residue extract over silica gel
(f) isolating of 2'-p-Hydroxy benzoyl mussaenosidic acid from the adsorbed extract by column chromatography

### Brief Description of the accompanying drawings

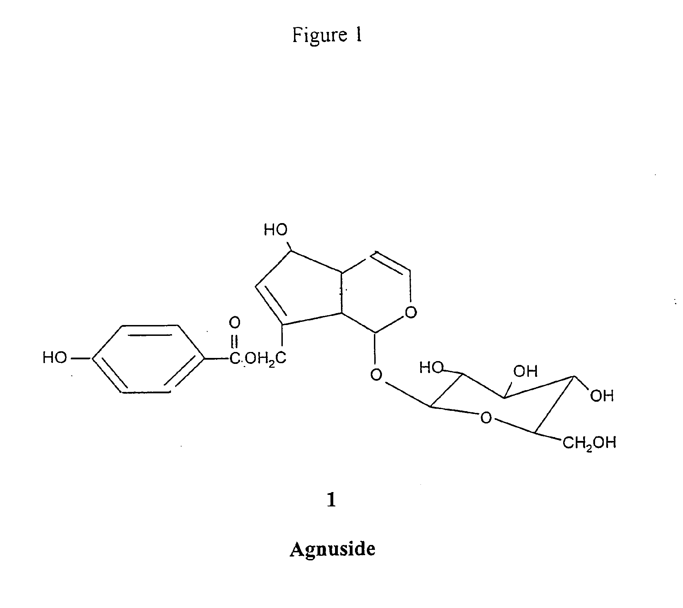

**Figure 1** represents the compound 10-O-p-hydroxybenzoylaucubin (Agnuside) of formula 1.

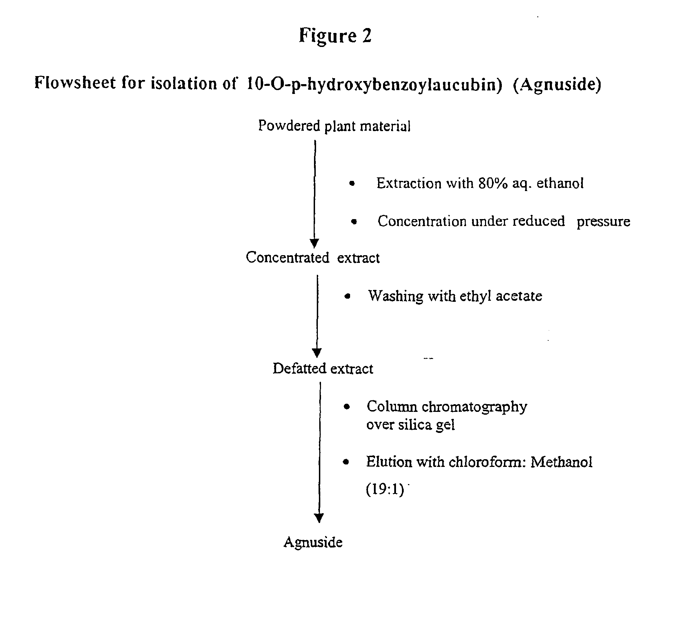

**Figure 2** represents flow-sheet for isolation of 10-O-p-hydroxybenzoylaucubin) (Agnuside).

The invention is described in detail by the examples given below which should not be construed to the limit of scope of the present invention

### Example 1

The shade dried and powdered leaves of (1 kg) *V. negundo* were extracted with 80% ethanol (4 x 3L) by percolation. The pooled extract was concentrated under reduced pressure at below 50°C to 1 litre of aqueous concentrate. The aqueous concentrate was washed with ethyl acetate (3 x 500 ml) and further concentrated to 400 ml. This syrupy residue was adsorbed over silica gel (400 g) and allowed to dry at room temperature. The slurry was put on silica gel column and eluted with mixture of chloroform-methanol (19: 1), furnished 1 (2.1g) (coded as 033 (1)

### Example 2

The finely ground leaves (200 g) of *Vitex negundo* were extracted thrice with petroleum ether (60-80°) (1 litre for the first extraction and 600 ml each for two subsequent x extractions) The drug was freed of solvent at room temperature. It was then extracted with ethanol (1 litre for the first extraction and then thrice with the same solvent 600 ml each time) Evaporation of ethanol in a rotary film evaporator yielded 30 g of extract (coded as

### Example 3

Treatment of experimental animals with the 033 and 033(1) against CCU induced liver damage reduced the elevated levels of serum GPT, GOT, ALP, bilirubin, TG and hepatic lipid peroxidation and increased the GSH level. A comparison with the known hepatoprotective agent silymarin revealed that 033 and 033(1) exhibited higher protective potential in most of the parameters with respect to their effect on elevated levels of above parameters by CCU The hepatoprotective activity observed with 033 and 033(1} was: serum GPT- 67.52 & 59.52, GOT- 58.91 & 57.42, ALP- 58.26 & 60.17, Bilirubin- 60.00 & 71.79, TG- 46.47 & 38.53 and in liver homogenate LP- 55.71 & 41.48 and GSH- 61.42 & 38.12% respectively. The same with silymarin was: 58.44, 51.63, 52.26, 57.50, 41.30, 62.05 & 60.15 respectively (Table-1). **Example 4**

Treatment of animals against the galactosamine (GaIN) induced hepatic damage also reduced the elevated levels of serum GPT, GOT, ALP, bilirubin, TG and hepatic lipid peroxidation and increased the GSH level. The hepatoprotective activity observed with 033 and 033(1) was 59.58 & 44.97, 56.62 & 44.05, 54.28 & 47.66, 57.71 & 66.66, 57.89 & 49.33 percent in serum GPT, GOT, Bilirubin, ALP, and TG respectively and 67.51 & 48.34, 66.56 & 55.13 percent in hepatic lipid peroxidation (LP) and GSH respectively. The same with silymarin was 57.38, 55.40, .60.00, 53.54,46.17, 69.59, 67.18 percent respectively (Table-2).

Advantages of the present invention over currently used plant based hepatoprotectives:
1. Agnuside is more potent than the commercially available herbal hepatoprotective agent silymarin.
2. Silymarin is a mixture of three constituents whose relative proportion varies from batch to batch while agnuside is a pure compound.

**Table 1: Hepatoprotective activity (in vivo) of 033, 033 (1) and Silymarin fed at 48h, 24h, 2h before and 6h after CCU (1 ml/kg, p.o.) induced hepatic injury in ratS³.**

| Treatment | Dose | Serum parameters | | | | | Hepatic parameters | |
|---|---|---|---|---|---|---|---|---|
| | mg/kg p.o. | OPT (Units) | GOT (Units) | A1.F | Bihrubm (mg %) | Triglycerides (mₛ %) | Lipid Peroxidation^{c} | Glutathione^{d} |
| 033+CCl₄ | 400 | 67.52 | 58.91 | 58.26 | 60.00 | 46.47 | 55.71 | 61.42 |
| 033(1)+ CCl₄ | 50 | 59.52 | 57.43 | 6017 | 71.79 | 38.53 | 41.48 | 38.12 |
| Silymarin + CCl₄ | 50 | 58.44 | 51.63 | 52.26 | 57.50 | 41.30 | 62.05 | 60.15 |

| | | | | | | | | |
|---|---|---|---|---|---|---|---|---|
| a: Values represent the mean percent hepatoprotective activity of six animals in each group. H: Hepatoprotective activity was calculated as {1-(T-V/C • V)} x 100 where'T' is mean value of drug and CCU, "C" is mean value of CCU alone and "V" is the mean value of vehicle treated animals. Unit: each unit is (mole pyruvate/min/L. b: is 11 mole of p-nitrophenol *formed*/*mini* U c: is n moles MDA/g liver., d: is n mole GSH/g liver | | | | | | | | |

**Table 2: Hepatoproteclive activity (m vivo) of 033,033 (1) and Silymarin fed at 48 h, 24h, 2h before and 6h after GalN (300 mg/kg, s.c.) induced hepatic injury in rats^{a}.**

| Treatment | Dose | Serum parameters | | | | | Hepatic parameters | |
|---|---|---|---|---|---|---|---|---|
| | mg/kg, p.o | GPT (Units) | GOT (Units) | Bilirubin (mg%) | ALP^{b} | Triglycerides (mg %) | Lipid peroxidation^{c} | Glulathi One^{d} |
| 033 + GaIN | 400 | 59.58 | 56.62 | 54.28 | 57.71 | 57.89 | 67.51 | 66.56 |
| 033(1) + GaIN | 50 | 44.97 | 44.05 | 47.66 | 66.66 | 49.33 | 48.34 | 55.13 |
| Silymarirri-GaIN | 50 | 57.38 | 55.40 | 60.00 | 53.54 | 46.17 | 69.59 | 67.18 |

| | | | | | | | | |
|---|---|---|---|---|---|---|---|---|
| a: Values represent the mean percent hepatoproteclive activity of six animals in each group. H: Hepatoprotective activity was calculated as {I- (T - V / C - V)} x 100 where "T" is mean value of drug and GaIN, 'C' is mean value of GaIN alone and "V" is the mean value of vehicle treated animals. Unit: each unit is pinole pyruvate/min/L. b: is M, mole of /j-nitrophenol formed/min/ L, c: is n moles MDA/g liver., d: is p. mole GSH/g liver., | | | | | | | | |

## Claims

1. Use of 10-O-p-hydroxybenzoylaucubin compound of Formula 1, also called Agnuside, optionally individual or in combination with one or more pharmaceutically acceptable additives for the manufacture of hepatoprotective agents for treating and/or preventing hepatic disease conditions in mammals including human beings.

2. The use as claimed in claim 1, wherein said agents reduce the elevated levels of serum glutamin-pyruvic transaminase (GPT) about 70%.

3. The use of claim 1, wherein the said agents reduce the elevated levels of serum glutamin-oxalo acetic transaminase (GOT) about 60%

4. The use as claimed in claim 1, wherein the said agents reduce the elevated levels of serum alkaline phosphatase (ALP) about 62%.

5. The use as claimed in claim 1, wherein the said agents reduce the elevated levels of serum tryglycerides about 70%.

6. The use as claimed in claim 1, wherein said agents reduce the elevated level of bilirubin about 72%.

7. The use as claimed in claim 1, wherein the said agnuside is obtained from the whole plant.

8. The use as claimed in claim 1, wherein 10-O-p-hydroxybenzoylaucubin is of concentration ranging between about 20 to 200 mg /kg-body weight.

9. The use as claimed in claim 1, wherein 10-O-p-hydroxybenzoylaucubin is of concentration is about 50 mg/kg-body weight.

10. The use as claimed in claim 1, wherein the pathological condition is selected from liver disorder.

11. The use as claimed in claim 1, wherein the subject is selected from mammals and animals preferably humans.

12. The use as claimed in claim 1, wherein said agents are used singly or in combination with pharmaceutically acceptable carriers.

13. The use according to claim 1 wherein said agents are administered to subject in combination with pharmaceutically acceptable additives, carriers, diluents, solvents, filters. Lubricants, excipients, binder or stabilizers.

14. The use as claimed in claim 1, wherein the desired dosage is administered for both preventive and curative properties.

15. The use as claimed in claim 1, wherein said agents are administered orally or by any clinically/medically accepted methods.

16. The use as claimed in claim 1, wherein the preferred dosage for human beings is about 5 mg/Kg of body weight.

17. 10-O-p-hydroxybenzoylaucubin compound of claim 1, available in powder, tablet, capsule, syrup, granules, emulsion, aerosoal, beads and the like for the use as hepatoprotective.

18. The use of 10-O-p-hydroxybenzoylaucubin compound as claimed in claim 1 for treating and/or preventing Liver cirrhosis, Galactosemia, Hemoanigoma, Hemochromatosis, Hepatitis A, Hepatitis B, Hepatitis C, Hepatitis D, Hepatitis E, Hepatitis G, Alcholic Liver disease, Autoimmune hepatitis, Cancer of Liver, Biliary Atresia, Glycogen Storage Disease 1, Alpha-1-antitrypsin deficiency, Alagille syndrome, Byler Disease, Caroli disease, Fatty liver, Itching in Liver, Primar Biliary Cirrhosis, Sclerosing Cholangitis or Protoporphyria Erythroepatic.

## Patentansprüche

1. Verwendung einer 10-O-p-Hydroxybenzoylaucubin-Verbindung der Formel 1, auch Agnusid genannt, optional individuell oder in Kombination mit einem oder mehreren pharmazeutisch annehmbaren Zusatzstoffen zur Fertigung von leberschützenden Mitteln zur Behandlung und/oder Vorbeugung von hepatischen Krankheitszuständen bei Säugern, einschließlich des Menschen.

2. Verwendung nach Anspruch 1, wobei die Mittel erhöhte Spiegel von Serum-Glutamat-Pyruvat-Transaminase (GPT) um etwa 70 % reduzieren.

3. Verwendung nach Anspruch 1, wobei die Mittel erhöhte Spiegel von Serum-Glutamat-Oxalacetat-Transaminase (GOT) um etwa 60 % reduzieren.

4. Verwendung nach Anspruch 1, wobei die Mittel erhöhte Spiegel von alkalischer Serum-Phosphatase (ALP) um etwa 62 % reduzieren.

5. Verwendung nach Anspruch 1, wobei die Mittel erhöhte Spiegel von Serum-Triglyceriden um etwa 70 % reduzieren.

6. Verwendung nach Anspruch 1, wobei die Mittel erhöhte Spiegel von Bilirubin um etwa 72 % reduzieren.

7. Verwendung nach Anspruch 1, wobei das Agnusid aus der Gesamtpflanze erhalten wird.

8. Verwendung nach Anspruch 1, wobei die Konzentration von 10-O-p-Hydroxybenzoylaucubin zwischen etwa 20 und 200 mg/kg-Körpergewicht variiert.

9. Verwendung nach Anspruch 1, wobei die Konzentration von 10-O-p-Hydroxybenzoylaucubin bei etwa 50 mg/kg-Körpergewicht liegt.

10. Verwendung nach Anspruch 1, wobei der pathologische Zustand ausgewählt ist aus einer Leberstörung.

11. Verwendung nach Anspruch 1, wobei das Subjekt ausgewählt ist aus Säugern und Tieren, bevorzugt Menschen.

12. Verwendung nach Anspruch 1, wobei die Mittel einzeln oder in Kombination mit pharmazeutisch annehmbaren Trägem verwendet werden.

13. Verwendung nach Anspruch 1, wobei die Mittel dem Subjekt in Kombination mit pharmazeutisch annehmbaren Zusatzstoffen, Trägern, Verdünnungsmitteln, Lösemitteln, Filtern, Gleitmitteln, Exzipienten, Bindemitteln oder Stabilisiermitteln verabreicht werden.

14. Verwendung nach Anspruch 1, wobei die gesuchte Dosierung sowohl aufgrund ihrer vorbeugenden als auch ihrer heilenden Eigenschaften verabreicht wird.

15. Verwendung nach Anspruch 1, wobei die Mittel oral oder mit jedem beliebigen klinisch/medizinisch annehmbaren Verfahren verabreicht werden.

16. Verwendung nach Anspruch 1, wobei die bevorzugte Dosierung für Menschen bei etwa 5 mg/kg Körpergewicht liegt.

17. 10-O-p-Hydroxybenzoylaucubin-Verbindung nach Anspruch 1, verfügbar als Pulver, Tablette, Kapsel, Sirup, Granulat, Emulsion, Aerosol oder Kügelchen und dergleichen zur Verwendung als Leberschutzmittel.

18. Verwendung der 10-O-p-Hydroxybenzoylaucubin-Verbindung nach Anspruch 1 zur Behandlung und/oder Vorbeugung von Leberzirrhose, Galaktosemie, Hämangiom, Hämochromatose, Hepatitis A, Hepatitis B, Hepatitis C, Hepatitis D, Hepatitis E, Hepatitis G, Alkoholleberkrankheit, Autoimmunhepatitis, Leberkrebs, biliäre Atresia, Glycogenspeicherkrankheit 1, Alpha-1-Antitrypsinmangel, Alagille-Syndrom, Byler-Krankheit, Caroli-Syndrom, Fettleber, Leber-Juckreiz, primäre biliäre Leberzirrhose, sklerosierende Cholangitis oder erythropoetische Proptoporphyrie.

## Revendications

1. Utilisation d'un composé de 10-O-p-hydroxybenzoylaucubine de formule 1, également dénommé agnuside, facultativement seul ou en combinaison avec un ou plusieurs additifs pharmaceutiquement acceptables pour la fabrication d'agents hépatoprotecteurs destinés au traitement et/ou à la prévention de maladies hépatiques chez les mammifères, y compris les êtres humains.

2. Utilisation selon la revendication 1, dans laquelle lesdits agents réduisent les niveaux élevés de transaminase glutamique pyruvique (GPT) sérique d'environ 70 %.

3. Utilisation selon la revendication 1, dans laquelle lesdits agents réduisent les niveaux élevés de transaminase glutamique-oxalo acétique (GOT) sérique d'environ 60%.

4. Utilisation selon la revendication 1, dans laquelle lesdits agents réduisent les niveaux élevés de phosphatase alcaline (ALP) sérique d'environ 62 %.

5. Utilisation selon la revendication 1, dans laquelle lesdits agents réduisent les niveaux élevés de triglycérides sériques d'environ 70 %.

6. Utilisation selon la revendication 1, dans laquelle lesdits agents réduisent le niveau élevé de bilirubine d'environ 72 %.

7. Utilisation selon la revendication 1, dans laquelle ledit agnuside est obtenu à partir de la plante totale.

8. Utilisation selon la revendication 1, dans laquelle la 10-O-p-hydroxybenzoylaucubine est présente en une concentration se trouvant dans la plage allant d'environ 20 à 200 mg/kg de poids corporel.

9. Utilisation selon la revendication 1, dans laquelle la 10-O-p-hydroxybenzoylaucubine est présente en une concentration d'environ 50 mg/kg de poids corporel.

10. Utilisation selon la revendication 1, dans laquelle la maladie est choisie parmi les troubles hépatiques.

11. Utilisation selon la revendication 1, dans laquelle le sujet est choisi parmi les mammifères et les animaux, de préférence les êtres humains.

12. Utilisation selon la revendication 1, dans laquelle lesdits agents sont utilisés seuls ou en combinaison avec des vecteurs pharmaceutiquement acceptables.

13. Utilisation selon la revendication 1, dans laquelle lesdits agents sont administrés à un sujet en combinaison avec des additifs, des vecteurs, des diluants, des solvants, des filtres, des lubrifiants, des excipients, des liants ou des stabilisants pharmaceutiquement acceptables.

14. Utilisation selon la revendication 1, dans laquelle la dose souhaitée est administrée pour ses propriétés préventives et ses propriétés curatives.

15. Utilisation selon la revendication 1, dans laquelle lesdits agents sont administrés, par la voie orale ou par tout procédé accepté sur le plan clinique/médical.

16. Utilisation selon la revendication 1, dans laquelle la dose préférée pour les êtres humains est d'environ 5 mg/kg de poids corporel.

17. Composé 10-O-p-hydroxybenzoylaucubine selon la revendication 1, disponible sous la forme d'une poudre, d'un comprimé, d'une gélule, d'un sirop, de granules, d'une émulsion, d'un aérosol, de billes et équivalents, destiné à être utilisé en tant qu'un agent hépatoprotecteur.

18. Utilisation du composé de 10-O-p-hydroxybenzoylaucubine selon la revendication 1, destiné au traitement et/ou à la prévention de la cirrhose hépatique, de la galactosémie, d'un hémangiome, de l'hémochromatose, de l'hépatite A, de l'hépatite B, de l'hépatite C, de l'hépatite D, de l'hépatite E, de l'hépatite G, d'une maladie hépatique due à l'alcool, d'une hépatite autoimmune, d'un cancer du foie, de l'atrésie biliaire, d'une maladie de stockage du glycogène 1, d'une déficience en alpha-1 antitrypsine, du syndrome d'Alagille, de la maladie de Byler, de la maladie de Caroli, de la stéatose hépatique, d'une démangeaison hépatique, de la cirrhose biliaire primaire, de la cholangite sclérosante ou de la protoporphyrie érythrohépatique.
